# EUROPÄISCHE PATENTSCHRIFT

(11) **EP 2 928 482 B1**
(45) Veröffentlichungstag und Bekanntmachung des Hinweises auf die Patenterteilung: **19.09.2018**
(21) Anmeldenummer: 13801557.3
(22) Anmeldetag: 04.12.2013
(51) Int. Cl.: A61K 35/28, C12N 5/0775, C12N 5/0789

(54) **VERBESSERUNG DER ZIELFINDUNGSKAPAZITÄT VON STAMMZELLEN**
IMPROVEMENT OF TARGETTING CAPACITY OF STEM CELLS
AMELIORATION DE LA CAPACITE DE CIBLAGE DE CELLULES SOUCHES

(30) Priorität: 06.12.2012 DE 102012111891
(43) Veröffentlichungstag der Anmeldung: 14.10.2015
(73) Patentinhaber: Eberhard Karls Universität Tübingen Medizinische Fakultät, 72074 Tübingen (DE)
(72) Erfinder: SCHAEFER, Richard, 65527 Niedernhausen (DE); DANIELYAN, Lusine, 72076 Tuebingen (DE); KEHLBACH, Rainer, 72119 Ammerbuch (DE); BANTLEON, Ruediger, 72639 Neuffen (DE); SIEGEL, Georg, 72074 Tuebingen (DE); VON AMELN-MAYERHOFER, Andreas, 72138 Rottenburg (DE); WENDEL, Hans-Peter, 72336 Balingen (DE); KLUBA, Torsten, 72127 Kusterdingen (DE); DOMINICI, Massimo, 41100 Modena (IT); CLAUSSEN, Claus, 72076 Tuebingen (DE); NORTHOFF, Hinnak, 72076 Tuebingen (DE)
(74) Vertreter: Witte, Weller & Partner Patentanwälte mbB
(86) Internationale Anmeldenummer: PCT/EP2013/075505
(87) Internationale Veröffentlichungsnummer: WO 2014/086839

(56) Entgegenhaltungen:
- DE-A1-102006 037 702
- DE-T2- 69 535 540
- SCHÄFER RICHARD ET AL: "Functional investigations on human mesenchymal stem cells exposed to magnetic fields and labeled with clinically approved iron nanoparticles", BMC CELL BIOLOGY, BIOMED CENTRAL, LONDON, GB, Bd. 11, Nr. 1, 6. April 2010 (2010-04-06), Seite 22, XP021072020, ISSN: 1471-2121, DOI: 10.1186/1471-2121-11-22
- I. M. BARBASH: "Systemic Delivery of Bone Marrow-Derived Mesenchymal Stem Cells to the Infarcted Myocardium: Feasibility, Cell Migration, and Body Distribution", CIRCULATION, Bd. 108, Nr. 7, 4. August 2003 (2003-08-04), Seiten 863-868, XP055099940, ISSN: 0009-7322, DOI: 10.1161/01.CIR.0000084828.50310.6A
- JOHN A. SCHOENHARD ET AL: "Stem Cell Therapy: Pieces of the Puzzle", JOURNAL OF CARDIOVASCULAR TRANSLATIONAL RESEARCH, Bd. 3, Nr. 1, 19. November 2009 (2009-11-19), Seiten 49-60, XP055100139, ISSN: 1937-5387, DOI: 10.1007/s12265-009-9148-z
- G. N. JONES ET AL: "Upregulating CXCR4 in Human Fetal Mesenchymal Stem Cells Enhances Engraftment and Bone Mechanics in a Mouse Model of Osteogenesis Imperfecta", STEM CELLS TRANSLATIONAL MEDICINE, Bd. 1, Nr. 1, 7. Dezember 2011 (2011-12-07), Seiten 70-78, XP055098907, ISSN: 2157-6564, DOI: 10.5966/sctm.2011-0007
- SACKSTEIN ROBERT ET AL: "Ex vivo glycan engineering of CD44 programs human multipotent mesenchymal stromal cell trafficking to bone", NATURE MEDICINE, NATURE PUBLISHING GROUP, NEW YORK, NY, US, Bd. 14, Nr. 2, 1. Februar 2008 (2008-02-01), Seiten 181-187, XP002526137, ISSN: 1078-8956, DOI: 10.1038/NM1703 [gefunden am 2008-01-13] in der Anmeldung erwähnt
- S. KUMAR ET AL: "Bone homing of mesenchymal stem cells by ectopic 4 integrin expression", THE FASEB JOURNAL, Bd. 21, Nr. 14, 20. Juli 2007 (2007-07-20) , Seiten 3917-3927, XP055099889, ISSN: 0892-6638, DOI: 10.1096/fj.07-8275com in der Anmeldung erwähnt
- ZHAOKANG CHENG ET AL: "Targeted Migration of Mesenchymal Stem Cells Modified With CXCR4 Gene to Infarcted Myocardium Improves Cardiac Performance", MOLECULAR THERAPY, Bd. 16, Nr. 3, 5. Februar 2008 (2008-02-05), Seiten 571-579, XP055099886, ISSN: 1525-0016, DOI: 10.1038/sj.mt.6300374 in der Anmeldung erwähnt
- GHADGE S K ET AL: "SDF-1alpha as a therapeutic stem cell homing factor in myocardial infarction", PHARMACOLOGY AND THERAPEUTICS, ELSEVIER, GB, Bd. 129, Nr. 1, 1. Januar 2011 (2011-01-01), Seiten 97-108, XP027562601, ISSN: 0163-7258 [gefunden am 2010-10-20]
- DEBANJAN SARKAR ET AL: "Chemical Engineering of Mesenchymal Stem Cells to Induce a Cell Rolling Response", BIOCONJUGATE CHEMISTRY, Bd. 19, Nr. 11, 19. November 2008 (2008-11-19), Seiten 2105-2109, XP055100140, ISSN: 1043-1802, DOI: 10.1021/bc800345q
- GAO J ET AL: "THE DYNAMIC IN VIVO DISTRIBUTION OF BONE MARROW-DERIVED MESENCHYMAL STEM CELLS AFTER INFUSION", CELLS TISSUES ORGANS, KARGER, BASEL, CH, Bd. 169, 1. Januar 2001 (2001-01-01), Seiten 12-20, XP009026035, ISSN: 1422-6405, DOI: 10.1159/000047856
- NOORT WILLY A ET AL: "Mesenchymal stem cells promote engraftment of human umbilical cord blood-derived CD34+ cells in NOD/SCID mice", EXPERIMENTAL HEMATOLOGY, ELSEVIER INC, US, Bd. 30, Nr. 8, 1. August 2002 (2002-08-01) , Seiten 870-878, XP002443447, ISSN: 0301-472X, DOI: 10.1016/S0301-472X(02)00820-2
- DEAN PHILIP JOHN KAVANAGH ET AL: "Hematopoietic Stem Cell Homing to Injured Tissues", STEM CELL REVIEWS AND REPORTS, HUMANA PRESS INC, NEW YORK, Bd. 7, Nr. 3, 22. Februar 2011 (2011-02-22), Seiten 672-682, XP019927868, ISSN: 1558-6804, DOI: 10.1007/S12015-011-9240-Z
- CHAVAKIS EMMANOUIL ET AL: "Role of beta2-integrins for homing and neovascularization capacity of endothelial progenitor cells", THE JOURNAL OF EXPERIMENTAL MEDICINE, ROCKEFELLER UNIVERSITY PRESS, US, Bd. 201, Nr. 1, 3. Januar 2005 (2005-01-03), Seiten 63-72, XP002487212, ISSN: 0022-1007, DOI: 10.1084/JEM.20041402
- DANIEL L J THOREK ET AL: "Superparamagnetic Iron Oxide Nanoparticle Probes for Molecular Imaging", ANNALS OF BIOMEDICAL ENGINEERING, KLUWER ACADEMIC PUBLISHERS-PLENUM PUBLISHERS, NE, Bd. 34, Nr. 1, 1. Januar 2006 (2006-01-01) , Seiten 23-38, XP019273025, ISSN: 1573-9686
- MERKEL O M ET AL: "Stability of siRNA polyplexes from poly(ethylenimine) and poly(ethylenimine)-g-poly(ethylene glycol) under in vivo conditions: Effects on pharmacokinetics and biodistribution measured by Fluorescence Fluctuation Spectroscopy and Single Photon Emission Computed Tomography (SPECT) imaging", JOURNAL OF CONTROLLED RELEASE, ELSEVIER, AMSTERDAM, NL, Bd. 138, Nr. 2, 1. September 2009 (2009-09-01), Seiten 148-159, XP026394867, ISSN: 0168-3659 [gefunden am 2009-05-19]

## Beschreibung

Die vorliegende Erfindung betrifft ein Verfahren zur Verbesserung der Zielfindungskapazität von mesenchymalen Stammzellen, eine Präparation, die entsprechend verbesserte mesenchymalen Stammzellen aufweist, sowie die Verwendung eines linearen kationischen Polymers zur Verbesserung der Zielfindungskapazität von mesenchymalen Stammzellen.

Stammzellen, insbesondere mesenchymale Stammzellen (MSCs), sind aufgrund ihres regenerativen und immunmodulatorischen Potentials vielversprechende Kandidaten für eine sog. zelluläre Therapie zur Behandlung verschiedener Krankheiten. Beispiele sind die Transplantat-Wirt-Krankheit und Osteogenesis imperfecta. Die zelluläre Therapie erfordert das zuverlässige und ausreichende Zuführen von Zellen zu der gewünschten anatomischen Struktur sowie das Überleben des zellulären Transplantates.

Systemisch, bzw. intravenös zugeführte MSCs sind auf die Stelle der Schädigung ausgerichtet. Allerdings akkumuliert der Großteil der transplantierten MSCs durch einen First-Pass-Effekt in ungewünschten Lokalisierungen, insbesondere in parenchymalen Organen wie der Leber, Milz und Lunge. Die Mechanismen, die der Akkumulierung von MSCs in nicht-verletzten parenchymalen Organen nach der intravaskulären Verabreichung unterliegen, sind noch nicht vollständig verstanden. Es wird angenommen, dass die massive Präsenz von transplantierten MSCs in der Lunge das Ergebnis eines unspezifischen Einfangprozesses der relativ großen MSCs in den kleinen pulmonalen Gefäßen ist. Es scheinen jedoch auch spezifischere Mechanismen die Adhäsion von MSCs an das pulmonale Epithel zu vermitteln.

Die günstigen Wirkungen der MSCs werden durch parakrine Faktoren vermittelt, die von den MSCs freigesetzt werden. Allerdings ist die genaue Transplantationsdosis für die MSCs, die an der Stelle der Schädigung vorzuherrschen hat, nach wie vor unklar. Eine Voraussetzung für das Auffinden der adäquaten Transplantationsdosis ist die direkte Zufuhr der MSCs zu der Zielregion. Ferner kann die ungerichtete Akkumulierung der transplantierten MSCs in verschiedenen Organen und Geweben das Risiko von Nebenwirkungen erhöhen und die therapeutischen Wirkungen der MSCs an der Stelle der Schädigung reduzieren. Dies ist von besonderer Bedeutung, da gezeigt wurde, dass MSCs nach systemischer Verabreichung zu neoplastischen Geweben migrieren und zum Tumorwachstum beitragen sowie das Wachstum von Metastasen fördern.

Die Interaktion von MSCs mit dem Endothel des Wirtes ist ein entscheidender Faktor für die Zielfindung der MSCs nach deren Applikation in das Gefäßsystem. Die komplexen Prozesse, die zu der Adhäsion der Zellen an das Endothel und der transendothelialen Migration beitragen, welche in der Zielfindung an der Stelle der Verletzung resultiert, erfordern eine empfindliche Balance von Chemokinrezeptoren und Adhäsionsmolekülen. Humane MSCs exprimieren eine Vielzahl von Chemokinrezeptoren, die zu den CC- und CXC-Familien gehören, sowie Adhäsionsmolekülen, wie Integrine, Endoglin (CD105), VCAM-1 (CD106) und H-CAM (CD44). Die Adhäsion von MSCs an das Endothel kann unter entzündlichen und nicht entzündlichen Bedingungen beobachtet werden, wobei die Gegenwart von proentzündlichen Zytokinen, wie TNF-α, oder Chemokinen und deren Liganden wie SDF-1a, die Adhäsion und transendotheliale Migration von MSCs fördern.

Im Stand der Technik wurden verschiedene Strategien entwickelt, um die Zielfindungskapazität von MSCs zu optimieren. Cheng et al. (2008), Targeted migration of mesenchymal stem cells modified with CXCR4 gene to infarcted myocardium improves cardiac performance, Mol. Ther. 16 (3), Seiten 571-579, schlagen hierzu die Erhöhung der Expression von CXCR4 vor. Kumar und Ponnazhagan (2007), Bone homing of mesenchymal stem cells by ectopic alpha 4 Integrin expression, The FASEB Journal 21 (14), Seiten 3917-3927, schlagen die Erhöhung der Expression von VLA-4 (CD49d) vor. Sackstein et al. (2008), Ex vivo glycan engineering of CD44 programs human multipotent mesenchymal stromal cell trafficking to bone, Nat. Med. 14(2), Seiten 181 bis 187, schlagen die Manipulation der Expression von HCELL vor. Sarkar et al. (2011), Engineered cell homing, Blood 118 (25), e184-191, beschreiben die Manipulation von PSGL-1.

Die im Stand der Technik beschriebenen Technologien erfordern aufwendige genetische Modifizierungen oder komplexe Konstruktionen *in vitro.* Ferner haben sich die vorgeschlagenen Lösungen aufgrund der ubiquitären Expression von Integrinen und Selektinen nicht bewährt.

Hui-Zhu et al. (2012) beschreiben die Markierung von mesenchymalen Stammzellen aus dem Knochenmark (BMSCs) durch Polythylenimin-superparamagnetisches Eisenoxid (PEI2k-SPI0) für das MR-Imaging.

In der DE 10 2006 037 702 wird die Inkubation von Stammzellen mit einem Magnetkomposit, das kationische Polymerverbindungen und darin eingekapselte Bioliganden aufweisen kann, beschrieben.

Barbash et al. (2003), Systemic Delivery of Bone Marrow-Derived Mesenchymal Stem Cells to the Infarcted Myocardium: Feasibility, Cell Migration, and Body Distribution", Circulation, Bd. 108, Nr. 7, Seiten 863-868, und Noort et al. (2002), Mesenchymal stem cells promote engraftment of human umbilical cord blood-derived CD34+ cells in NOD/SCID mice, Experimental Hematology, Elsevier Inc., US, Bd. 30, Nr. 8, 1, Seiten 870-878, beschreiben, dass bei systemischer Gabe von Stammzellen nur ein geringer Anteil der Zellen den gewünschten Wirkort erreicht. Viele Zellen bleiben in Lunge, Leber und Niere.

Eine Verringerung des Verbleibens vom MSC in der Lunge wurde durch Gefäßerweiterung erreicht; vgl. Barbash et al. (2003; a.a.O.) und Gao et al. (2001), The Dynamic In Vivo Distribution of Bone Marrow-Derived Mesenchymal Stem Cells After Infusion, Cells Tissues Organs, Karger, Basel, CH, Bd. 169, Seiten 12-20.

Es ist bekannt, dass die Rekrutierung von Stammzellen in verwundetem Gewebe oder Organen über die Ausschüttung von Chemokinen, Cytokinen und Wachstumsfaktoren gesteuert wird. Einer dieser Faktoren ist Stromal Derived Factor- 1 (SDF-1) welcher auf CXCR4+ Zellen wirkt; vgl. Schoenhard et al. (2009), Stem Cell Therapy: Pieces of the Puzzle, Journal of Cardiovascular Translational Research, Bd. 3, Nr. 1, Seiten 49-60.

Jones et al. (2011), Upregulating CXCR4 in Human Fetal Mesenchymal Stem Cells Enhances Engraftment and Bone Mechanics in a Mouse Model of Osteogenesis Imperfecta, Stem Cells Translational Medicine, Bd. 1, Nr. 1, Seiten 70-78, Cheng et al. (2008), Targeted Migration of Mesenchymal Stem Cells Modified With CXCR4 Gene to Infarcted Myocardium Improves Cardiac Performance, Molecular Therapy, Bd. 16, Nr. 3, Seiten 571-579, und Ghadge et al. (2011), SDF-1 Alpha as a Therapeutic Stem Cell Homing Factor in Myocardial Infarction, Pharmacology and Therapeutics, Elsevier, GB, Bd. 129, Nr. 1, Seiten 97-108, beschreiben eine erhöhte Rekrutierung von MSC in Knochen bzw Myokard durch die Stimulierung der CXCR4 Expression von MSC welche deren Migration erhöht.

Rekrutierung in Knochengewebe wurde auch durch erhöhte Expression von alpha4-integrin und CD44 in MSC erreicht; vgl. Kumar et al. (2007), Bone Homing of Mesenchymal Stem Cells by Ectopic 4 Integrin Expression, und Sackstein et al. (2008), Ex Vivo Glycan Engineering of CD44 Programs Human Multipotent Mesenchymal Stromal Cell Trafficking to Bone, Nature Medicine, Nature Publishing Group, New York, US, Bd. 14, Nr. 2, Seiten 181-187.

Bei der Zielfindung von humanen Stammzellen und endothelialen Vorläuferzellen spielen auch Integrine eine große Rolle; vgl. Kavanagh et al. (2011), Hematopoietic Stem Cell Homing to Injured Tissues, Stem Cell Reviews and Reports, Humana Press, Inc., New York, US, Bd. 7, Nr. 3, Seiten 672-682. Emmanouil et al. (2005), Role of Beta2-Integrins for Homing and Neovascularization Capacity of Endothelial Progenitor Cells, The Journal of Experimental Medicine, Rockefeller University Press, US, Bd. 201, Nr. 1, Seiten 63-72. Erhöhte Expression wurde durch die Einführung des gewünschten Gens oder eine Inkubation mit Faktoren wie SDF-1 oder mit aktivierenden Antikörpern erreicht. Es wurde auch versucht die Adhäsion von Zellen durch kovalente Modifizierung zu beeinflussen; vgl. Sarkar et al. (2008), Chemical Engineering of Mesenchymal Stem Cells to Induce a Cell Rolling Response, Bioconjugate Chemistry, Bd. 19, Nr. 11, Seiten 2105-2109.

Vor diesem Hintergrund ist es eine Aufgabe der vorliegenden Erfindung, ein Verfahren bereitzustellen, mit dem die Zielfindungskapazität von Stammzellen verbessert werden kann und die Nachteile aus dem Stand der Technik vermieden werden. Insbesondere soll ein solches Verfahren bereitgestellt werden, das keine komplexen genetischen Modifizierungen oder Technologien erfordert.

Diese Aufgabe wird durch ein Verfahren zur Verbesserung der Zielfindungskapazität von mesenchymalen Stammzellen (MSCs) gelöst, das folgende Schritte aufweist: (1) Bereitstellung von MSCs, und (2) Inkubation der MSCs mit einem linearen kationischen Agens.

Die Erfinder haben überraschenderweise festgestellt, dass eine einfache Behandlung von Stammzellen mit einem linearen kationischen Polymers die MSCs derart verändert, dass der First-Pass-Effekt signifikant vermindert und der Anteil der Zellen, die das Zielgebiet erreichen, deutlich erhöht wird.

Unter "Zielfindungskapazität" wird die Fähigkeit einer Stammzelle verstanden, entlang eines Konzentrationsgradienten zum Ort der höchsten Konzentration eines oder mehrerer Signalmoleküle zu migrieren. Ein Beispiel solcher Signalmoleküle bildet die Gruppe der Chemokine. Eine erfindungsgemäß besonders relevante Form der Zielfindungskapazität ist die Fähigkeit einer Stammzelle, nach einer Verabreichung in einen Organismus an den Ort einer zellulären Schädigung oder eines Gewebeschadens zu migrieren.

Die der Erfindung zugrundeliegende Aufgabe wird hiermit vollkommen gelöst.

Anders als dies bspw. in der DE 10 2006 037 702 beschrieben ist, wird die Zielfindungskapazität der Stammzellen ohne die Vermittlung durch Zielstrukturerkennende Bioliganden, wie Antikörpern, verbessert.

Kationische Agentien, wie z.B. PEI werden zur Transfektion von Zellen verwendet; vgl. DE 695 35 540 T2. PEI wird auch getestet für die *in vivo* Gentherapie. Allerdings wurden Modifikationen von PEI vorgeschlagen, um den First-Pass Effekt zu verringern oder zu umgehen; vgl. Merkel et al. (2009). Schäfer et al. (2010), Functional Investigations on Human Mesenchymal Stem Cells Exposed to Magnetic Fields and Labeled With Clinically Approved Iron Nanoparticles, BMC Cell Biology, Biomed Central, London, GB, Bd. 11, Nr. 1, beschreiben die Verwendung von PEI, um Zellen mit magnetischen Nanopartikeln zu markieren. PEI ermöglicht die Aufnahme dieser Nanopartikel in die Zellen, welche dann im Körper mittels MRI nachgewiesen werden können. Ein Einfluss auf die Zielfindung der Zellen oder eine Beeinflussung der Adhäsion ist im Stand der Technik nicht bekannt. Es ist auch kein Verfahren bekannt, welches ein kationisches Agens, wie PEI, zur Verbesserung der Zielfindungskapazität verwendet.

Geeignet sind grundsätzlich MSCs aus beliebigen Säugetieren. MSCs werden im Stand der Technik bereits im Rahmen der Zelltherapie eingesetzt und weisen sich durch ihr regeneratives und immunmodulatorisches Potential aus. Durch das erfindungsgemäße Verfahren wird die Zielfindungskapazität dieser Zellpopulationen deutlich erhöht. Durch die Verringerung des First-Pass-Effekts können deutlich geringere Mengen von Zellen verabreicht werden, wodurch Nebenwirkungen signifikant vermindert werden können.

Die Verwendung eines linearen kationischen Polymers hat den Vorteil, dass nach Erkenntnissen der Erfinder besonders gute Ergebnisse erzielt werden. Beispiele für geeignete lineare kationische Polymere sind Polyethylenimin (PEI) oder kationische Click-Polymere.

Dabei ist es erfindungsgemäß bevorzugt, wenn das lineare kationische Polymer Polyethylenimin (PEI) ist.

Die Erfinder haben festgestellt, dass die Behandlung von Stammzellen mit Polyethylenimin (PEI) besonders gute Ergebnisse liefert. PEI ist kommerziell erhältlich und steht deshalb in großen Mengen. Ein geeignetes PEI-Produkt ist bspw. jetPEI™ der Firma Polypuls-transfection S.A., Illkirch, Frankreich.

Nach einer Weiterentwicklung des erfindungsgemäßen Verfahrens sind die MSCs humane MSCs.

Humane MSCs (hMSCs), die erfindungsgemäß behandelt wurden, eignen sich besonders gut für klinische Anwendungen im Menschen. Dies haben Jahrzehnte lange klinische Anwendungen bestätigt. Die Einbringung tierischer Substanzen in den Menschen wird so vermieden. Weiterhin ist bei Beibehaltung des humanen Systems die optimale Übertragbarkeit von *In*-*vitro*-Erkenntnissen über humane Stammzellen in die Patientenanwendung gewährleistet.

Nach einer bevorzugten Weiterentwicklung des erfindungsgemäßen Verfahrens werden in einem dritten Schritt die Stammzellen zusätzlich mit Eisenoxidpartikel behandelt bzw. inkubiert.

Nach Erkenntnissen der Erfinder führt die zusätzliche Behandlung mit Eisenoxidnanopartikeln zu einer weiteren Verbesserung der Zielfindungskapazität der Stammzellen. Die Erfinder haben festgestellt, dass durch diese Maßnahme die Eignung der Stammzellen für eine lokale Applikation, beispielsweise eine Injektion in bzw. um den Ort von zellulären Schäden oder Gewebeschäden, erhöht wird. Erfindungsgemäß können die Stammzellen gleichzeitig mit dem kationischen linearen Polymer und den Eisenoxidnanopartikeln behandelt werden. Die Stammzellen können jedoch auch zuerst mit dem kationischen linearen Polymer und anschließend mit den Eisenoxidnanopartikeln behandelt werden. Die MSCs können außerdem zunächst mit den Eisenoxidnanopartikeln inkubiert werden und anschließend mit dem kationischen linearen Polymers.

Nach einer bevorzugten Variante handelt es sich bei den EisenoxidNanopartikel um Ferucarbotran.

Diese Maßnahme hat den Vorteil, dass nach Erkenntnissen der Erfinder besonders gute Ergebnisse erzielt werden. Eisenoxidnanopartikel bzw. Ferucarbotran ist kommerziell erhältlich, beispielsweise in Form des Produktes "Resovist®" der Firma Schering AG, Berlin, Deutschland.

Dabei ist es erfindungsgemäß weiter bevorzugt, wenn die Verbesserung der Zielfindungskapazität durch Inhibition der Zelladhäsionsrezeptoren der MSCs erfolgt.

Diese Maßnahme hat den Vorteil, dass nach Erkenntnissen der Erfinder molekulare Strukturen auf der Oberfläche der MSCs derart modifiziert werden, sodass der First-Pass-Effekt inhibiert und dadurch die Zielfindungskapazität erhöht wird.

Nach einer bevorzugten Weiterentwicklung des erfindungsgemäßen Verfahrens, erfolgt die Verbesserung der Zielfindungskapazität durch Stimulation der Chemokinrezeptoren der MSCs.

Diese Maßnahme hat den Vorteil, dass Strukturen auf der Oberfläche der Stammzellen derart verändert werden, dass die Migration in Richtung der höchsten Konzentration des Signalmoleküls verbessert wird. "Stimulation" umfasst erfindungsgemäß sowohl eine Erhöhung der Expression als auch der Aktivität bzw. Affinität der Chemokinrezeptoren der MSCs.

Offenbart wird außerdem eine Präparation, die Stammzellen, vorzugsweise mesenchymale Stammzellen (MSCs) und/oder hämatopoetische Stammzellen (HSCs) aufweist, die nach dem erfindungsgemäßen Verfahren erhalten bzw. behandelt wurden. Bei der Präparation handelt es sich vorzugsweise um eine pharmazeutische Präparation, weiter vorzugsweise um eine solche zur Behandlung von zellulären Schäden und/oder Gewebeschäden. Bei den zellulären Schäden bzw. Gewebeschäden kann es sich um Entzündungen, degenerative Erkrankungen, tumoröse Erkrankungen und neuronale Läsionen handeln.

Wie die Erfinder feststellen konnten, eignet sich die Präparation bzw. pharmazeutische Präparation zur Behandlung von Schlaganfällen, Herzinfarkten, Herzinsuffizienz, Knochen-/Gelenkserkrankungen einschließlich rheumatischer Erkrankungen, Alzheimersche Demenz, Parkinsonsche Krankheit, Autoimmunerkrankungen einschließlich Multipler Sklerose.

Ein weiterer Gegenstand der vorliegenden Erfindung betrifft die Verwendung eines linearen kationischen Polymers, weiter bevorzugt eines Polyethylenimins (PEIs), zur Verbesserung der Zellfindungskapazität von mesenchymalen Stammzellen (MSCs).

Die Merkmale, Eigenschaften und Vorteile des erfindungsgemäßen Verfahrens gelten gleichermaßen für die erfindungsgemäße Verwendung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird anhand von Ausführungsbeispielen näher erläutert, aus denen sich weitere Vorteile, Eigenschaften und Merkmale der Erfindung ergeben. Die Ausführungsbeispiele dienen lediglich zur Illustration und schränken die Reichweite der Erfindung nicht ein. Dabei wird Bezug genommen auf die beigefügten Figuren, in denen Folgendes dargestellt ist:

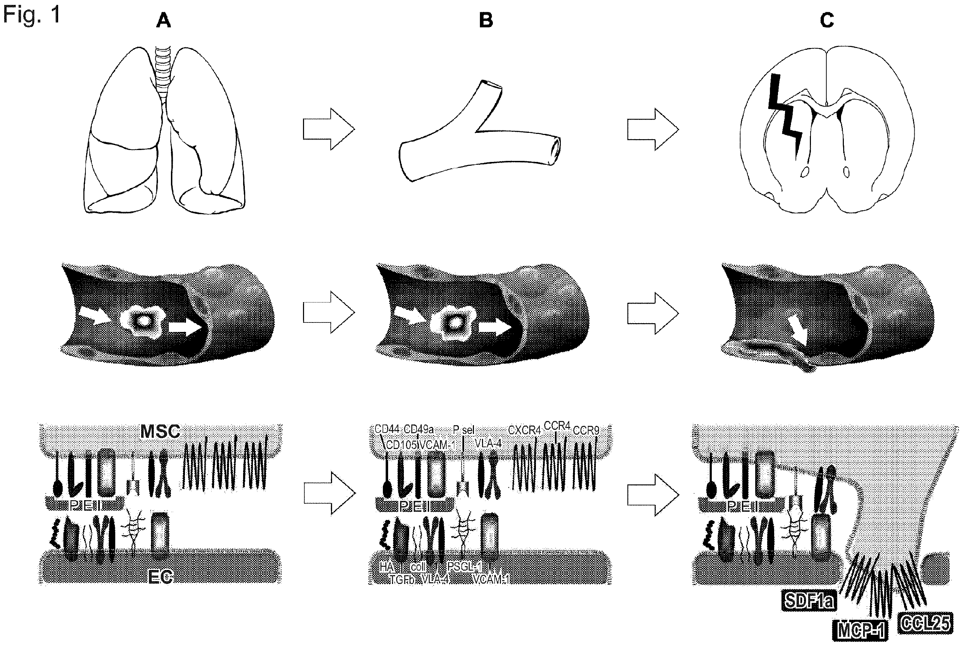

Fig.1 zeigt eine schematische Darstellung der Effekte der PEI-Behandlung von MSCs auf deren Homing-Rezeptorfunktion und Zielfindungskapazität. Die Funktion der Rezeptoren, welche die Adhäsion an Endothel in der Lunge (A) bzw. in nicht entzündlich verändertem Gewebe/Blutgefäßen (B) vermittelt, wird durch PEI blockiert. Hierdurch können die MSCs diese Bereiche ungehindert über den Blutstrom passieren. Im Zielgebiet (C) sind die Chemokine verstärkt exprimiert. Da PEI die Funktion der Chemokinrezeptoren nicht beeinträchtigt, können die MSCs dort ungehindert einwandern. In der Summe können mehr PEI-behandelte MSCs als unbehandelte MSCs in das Zielgebiet einwandern, da diese in geringerem Ausmaß in der Lunge bzw. in nicht entzündlich verändertem Gewebe/Blutgefäßen zurückgehalten werden

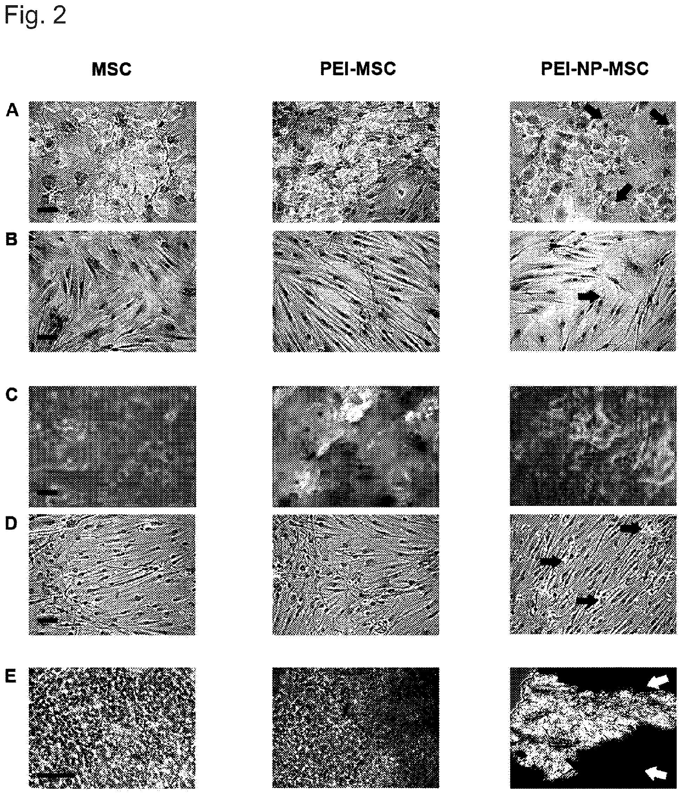

Fig. 2 zeigt die adipogene Red-Oil-O-Färbung von Lipidvakuolen [A], undifferenzierte Kontrolle [B], osteogene Alizarin-Rot-Färbung [C], undifferenzierte Kontrolle [D] und chondrogene (E) Differenzierungskapazität der unbehandelten MSCs, PEI-MSCs und PEI-NP-MSCs. Die Behandlung mit PEI mit/ohne Nanopartikel (NP) zeigt keinen Effekt auf die Differenzierungskapazität der MSCs. Pfeile: Komplexe von PEI+NP. Maßstabsbalken: 100 µm

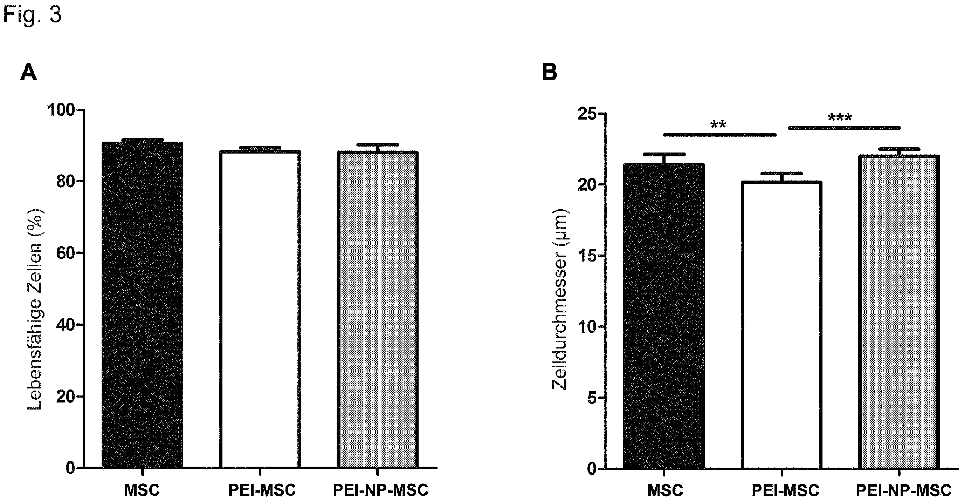

Fig. 3 zeigt die Viabilität der MSCs, welche durch die Behandlung mit PEI mit/ohne NP nicht beeinträchtigt ist (A). Die Behandlung mit PEI vermindert den Durchmesser der MSCs geringfügig aber signifikant (B).

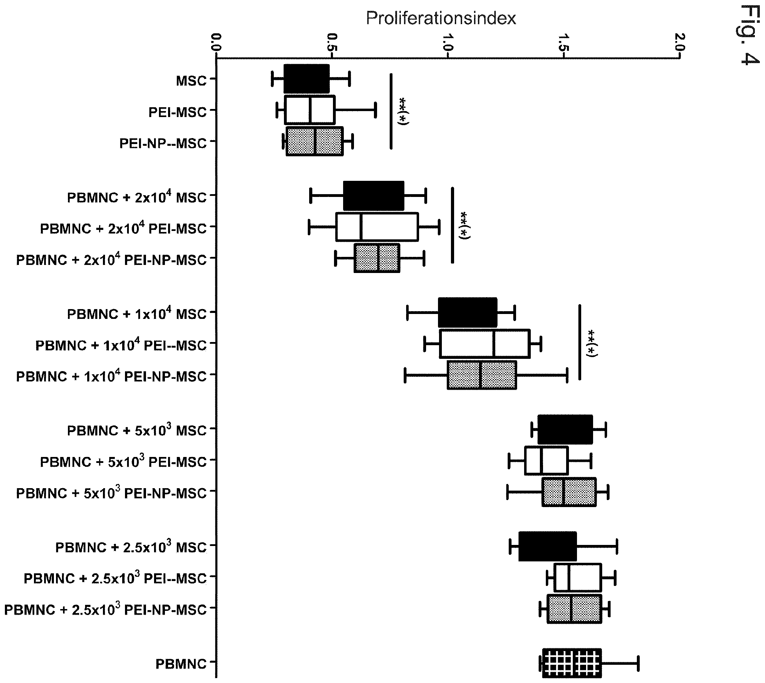

Fig. 4 zeigt die Potenz der MSCs zur Suppression der Proliferation von T-Zellen (*In*-*vitro*-Immunsuppression). Die Behandlung mit PEI mit/ohne NP zeigt keinen Effekt auf die immunsuppressiven Eigenschaften der MSCs.

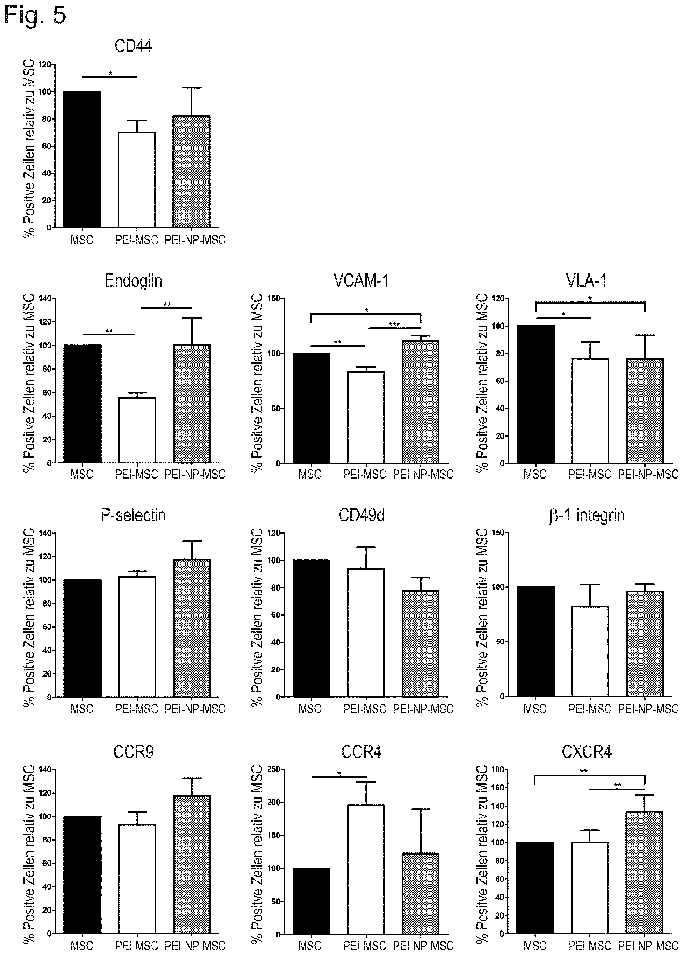

Fig. 5 zeigt die Expression/Zugänglichkeit von Homingrezeptoren und Chemokinrezeptoren von MSCs. Die Behandlung mit PEI vermindert die Expression/Zugänglichkeit von CD44, Endoglin, VCAM-1 und VLA-1, wohingegen die Chemokinrezeptoren CCR9 und CXCR4 unbeeinflusst bleiben und CCR4 sogar verstärkt exprimiert ist. Die Behandlung mit PEI+NP verstärkt die Expression von VCAM-1 und CXCR-4.

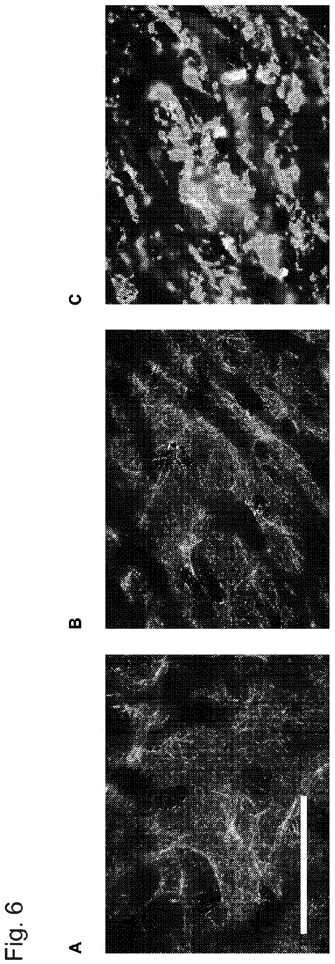

Fig. 6 zeigt die Expression von CXCR-4 auf MSCs. Im Vergleich zu unbehandelten MSCs (A) und PEI-MSCs (B) führt die Behandlung mit PEI+NP zu einer verstärkten Expression von CXCR-4 auf den MSCs (C). CXCR-4: grün, Zellkerne: blau (DAPI); Maßstabsbalken: 50 µm.

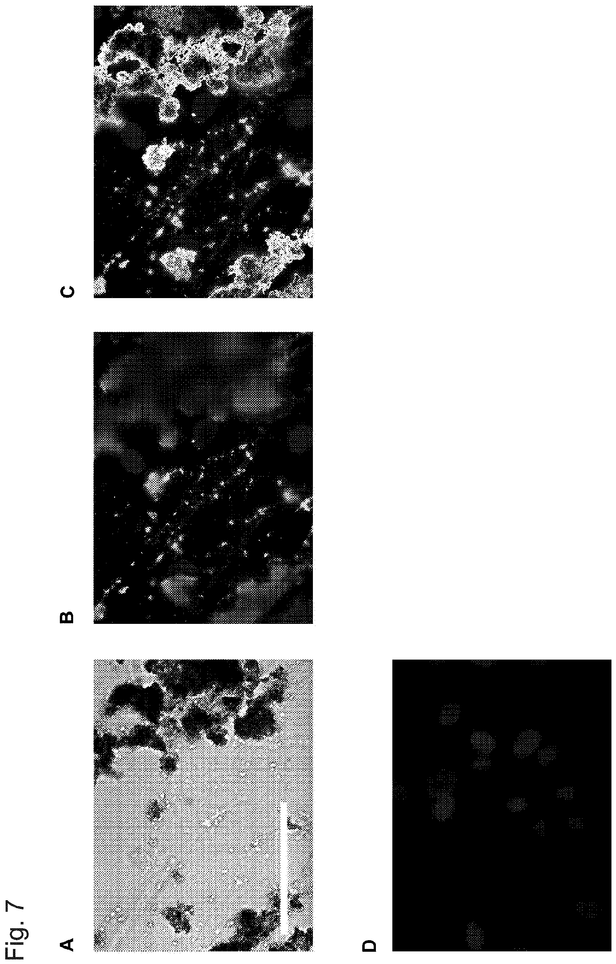

Fig. 7 zeigt die Expression von CXCR-4 auf PEI-NP-MSCs in Bezug zu den PEI-NP-Komplexen. Die verstärkte CXCR-4-Expression ist v.a. in der Nähe zu den extrazellulären PEI-NP-Komplexen zu beobachten. Die PEI-NP-Komplexe sind in der Phasenkontrast-Durchlichtmikroskopie als graue Agglomerate identifizierbar (A). Verstärkte CXCR-4-Expression im Bereich der PEI-NP-Komplexe (B, C). Die PEI-NP-Komplexe sind in der Überlagerungsdarstellung von A und B mittels Bearbeitung mittels "Adobe Photoshop CS5.1" in Graustufen hervorgehoben. Die CXCR-4-lsotyp-Kontrolle zeigt keine unspezifische Fluoreszenz im Bereich der PEI-NP-Komplexe (D). CXCR-4: grün, Zellkerne: blau (DAPI); Maßstabsbalken: 50 µm.

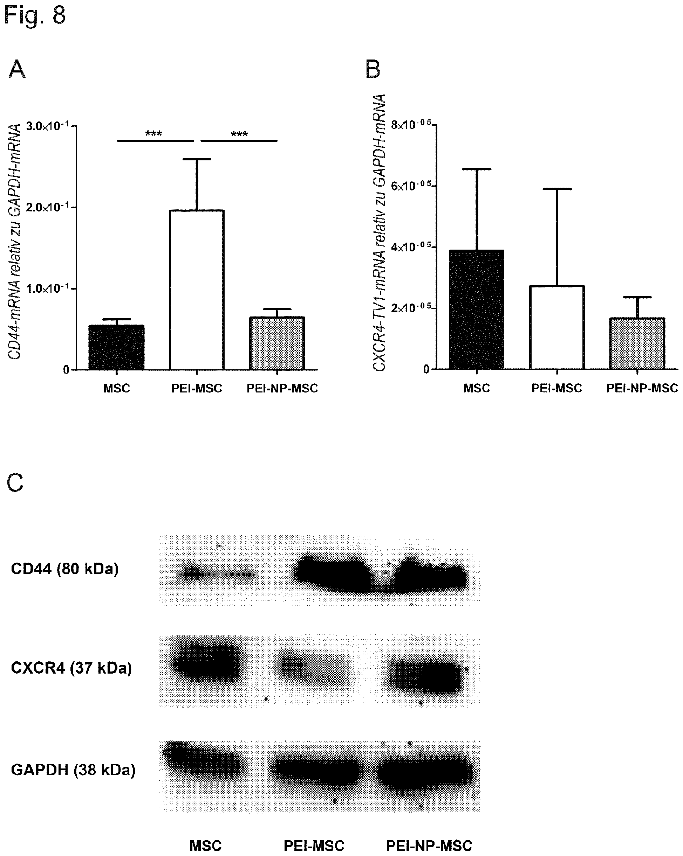

Fig. 8 zeigt Untersuchungen zur Regulation der CD44- bzw. CXCR4-Expression. Die Behandlung mit PEI führt zu einer verstärkten Expression von *CD44* mRNA (A) und CD44 Gesamtprotein (B) sowie zu einer verminderten Expression von CXCR-4 Gesamtprotein (C). Die Behandlung mit PEI+NP zeigt keinen Effekt auf die CD44- und CXCR-4-Expression, weder auf mRNA- noch Protein-Ebene (A-C).

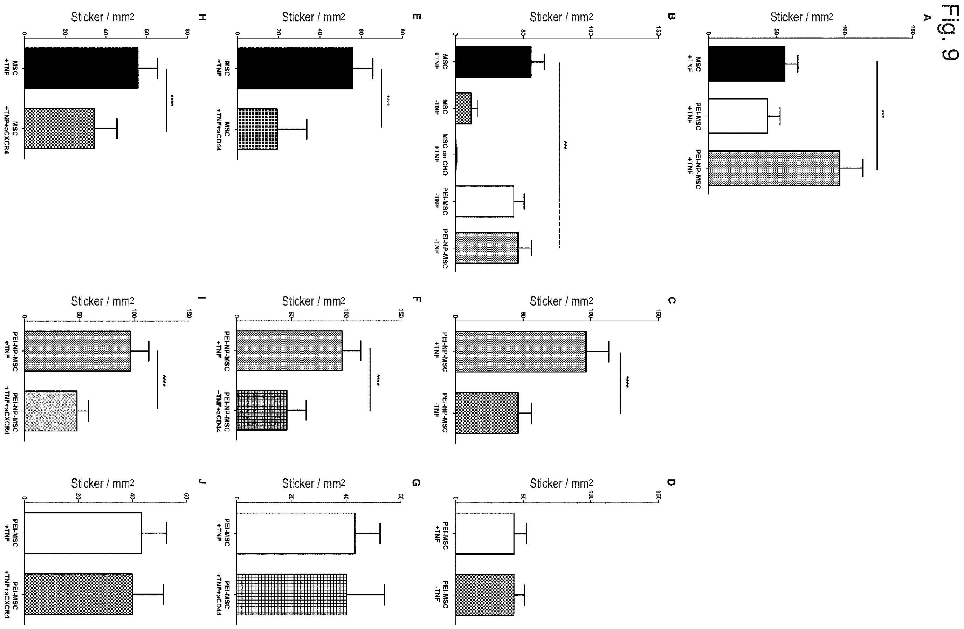

Fig. 9 zeigt das Adhäsionsverhalten der MSCs an TNF-α aktivierte bzw. nicht-aktivierte Endothelzellen im dynamischen Flusskammermodell *in vitro.* PEI-MSC zeigen eine verminderte Adhäsion an TNF-α-aktivierte Endothelzellen, wohingegen PEI-NP-MSCs eine verstärkte Adhäsion an TNF-α-aktivierte Endothelzellen zeigen (A). Behandelte und unbehandelte MSCs zeigen ein vermindertes Adhäsionsverhalten an nicht-aktivierte Endothelzellen in Vergleich zu unbehandelten MSCs an TNF-α-aktivierte Endothelzellen. Das Adhäsionsverhalten von PEI-MSCs und PEI-NP-MSCs an nicht-aktivierte Endothelzellen ist stärker als das Adhäsionsverhalten von unbehandelten MSCs an nicht-aktivierte Endothelzellen (B). Die verstärkte Adhäsion an TNF-α-aktivierte Endothelzellen nach Behandlung mit PEI+NP ist bei nicht-aktivierten Endothelzellen aufgehoben (C). Die verminderte Adhäsion nach Behandlung mit PEI ist unabhängig vom Aktivierungsgrad der Endothelzellen (D). Die Adhäsion von unbehandelten MSCs und PEI-NP-MSCs an TNF-α-aktivierte Endothelzellen kann mittels blockierenden anti-CD44 und anti-CXCR-4-Antikörpern vermindert werden (E, F, H, I), wohingegen die verminderte Adhäsion von PEI-MSCs nicht weiter mit blockierenden anti-CD44 und anti-CXCR-4-Antikörpern vermindert werden kann (G, J).

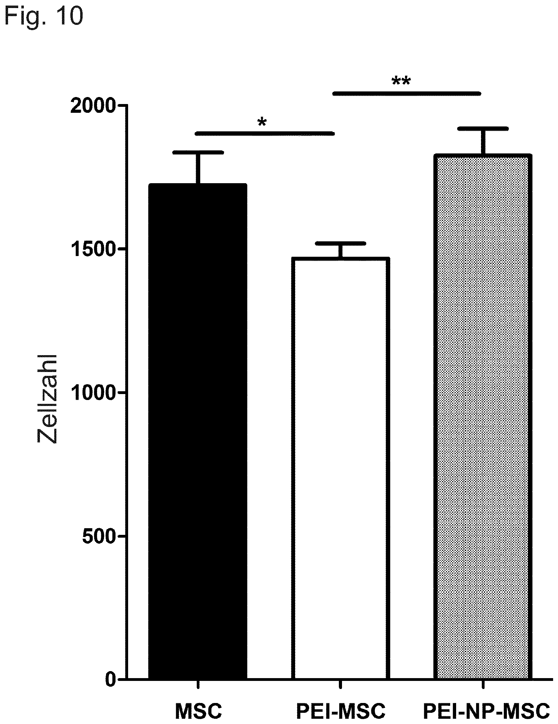

Fig. 10 zeigt die immunhistologische Quantifizierung von GFP⁺ MSCs in der Lunge 24h nach i.v. Transplantation *in vivo.* Die Behandlung mit PEI führt zu einer verminderten Detektion von MSCs in der Lunge.

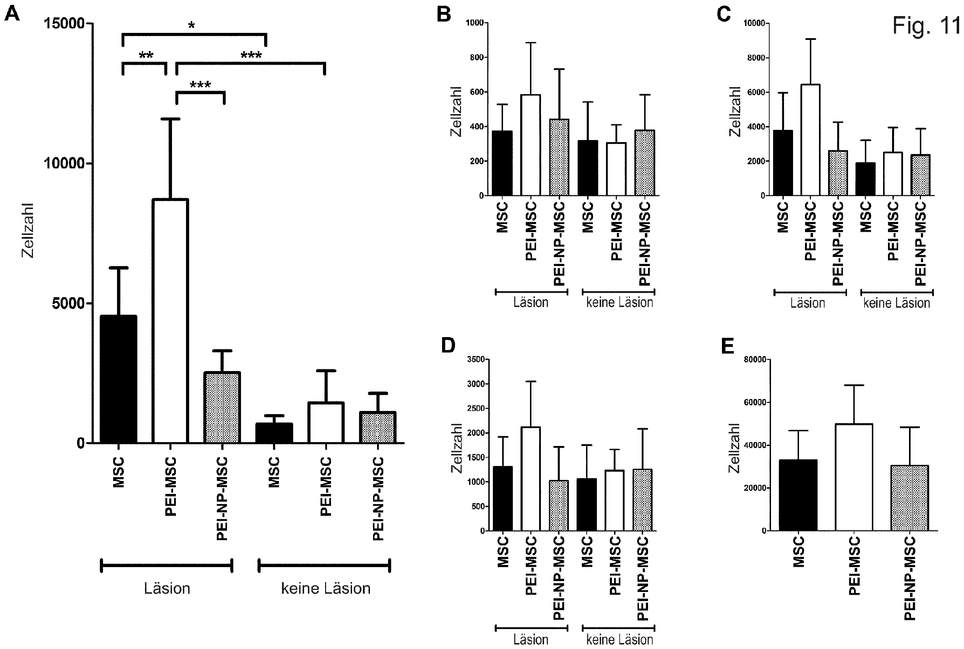

Fig. 11 zeigt die immunhistologische Quantifizierung von GFP⁺ MSCs im Gehirn 24h nach i.v. Transplantation in vivo. In der hippocampalen Hirnläsion waren mehr unbehandelte MSCs und PEI-MSCs im Vergleich zum nicht-lädierten, kontralateralen Hippocampus detektierbar, wobei im Bereich der Läsion mehr PEI-MSCs nachweisbar waren (A). In den anderen Hirnregionen (laterale Ventrikel [B], Hirnrinde [C], Striatum [D]) sowie im Gesamthirn (E) zeigte sich kein Unterschied zwischen den Hemisphären bzw. den Zellgruppen.

### Ausführungsbeispiele

### 1. Material und Methoden

### 1.1 Knochenmarkspräparation

Das Protokoll wurde von den lokalen zuständigen Behörden genehmigt. Knochenmark (BM) wurde unter sterilen Bedingungen während orthopädischen Operationen randomisiert gewählten Spendern entnommen, die nach entsprechender Aufklärung ihre schriftliche Zustimmung erteilt hatten. Von jedem Spender wurden 5-10 ml Gesamt-BM in einer sterilen Spritze gesammelt.

### 1.2 Isolierung von BM-MCS und Zellkultur

Um MSCs aus Gesamt-BM zu isolieren, wurden im Stand der Technik bekannte Technologien verwendet. Zusammengefasst, mononukleare Zellen aus BM wurden mittels Dichtegradientenzentrifugation auf Lymphoflot (Biotest, Dreieich, Deutschland) isoliert, zweimal mit PBS (Lonza, Walkersville, USA) gewaschen, gezählt und mit einer Dichte von 1x10⁵-Zellen/cm² in Medium in Zellkulturflaschen (Nunc, Roskilde, Dänemark) gegeben, das α-MEM (Lonza), 1% Penicillin-Streptomicin (Lonza) und 10% FBS (Lonza, Schweiz) enthielt. Die resultierenden passagierten Zellen wurden bei 37°C in feuchter Atmosphäre mit 5% CO₂ kultiviert. Die nicht-adhärenten Zellen wurden durch Waschvorgänge nach 24 Stunden entfernt und das Medium wurde zweimal die Woche gewechselt. Nach Erreichen der Subkonfluenz, wurden die MSCs unter Verwendung von Trypsin-EDTA (Lonza) abgelöst, mit einem CASY®-Zellzählgerät (Roche, Basel, Schweiz) gezählt und in frische Gewebekulturflaschen für die nächste Passage (P1) mit einer Dichte von 1000 Zellen pro cm² ausplattiert.

### 1.3 Transduktion mit grünem fluoreszierendem Protein (GFP)

GPF⁺-hBM-MSCs wurden freundlicherweise von M. Dominici, Modena, Italien, bereitgestellt. Es wurde ein bicistronischer retroviraler Vektor, der sich von einem murinen Stammzellvirus ableitet (pMIGR1), welcher für GFP codiert, verwendet, um MSC stabil genetisch zu modifizieren. Die retrovirale Produktion erfolgte durch FLYRD18-verpackende Zelllinien. MSCs wurden durch virusenthaltendes Medium von FLYRD18-GFP (beides ≈ 1x10⁶ TU/ml) transduziert. Die anschließende Kultivierung und Expansion von GFP⁺ hBM-MSCs erfolgte unter den folgenden Bedingungen: Die MSCs wurden mit einer Dichte von 5000 Zellen/cm² in Gewebekulturflaschen mit Standardkulturmedium ausplattiert, das DMEM mit niedriger Glukose (Gibco) enthielt, welches mit 10% FCS (Hyclone, Thermo Scientific) versetzt war. Das Kulturmedium wurde zweimal die Woche gewechselt. Nach Erreichen der Subkonfluenz, wurden die Zellen entweder subkultiviert, unmittelbar für Experimente verwendet oder als Stamm für spätere Experimente kryokonserviert.

### 1.4 Charakterisierung von MSCs durch Differenzierung in vitro und durchflusszytometrische Analyse

MSCs können funktionell durch ihre Differenzierungskapazität *in vitro* charakterisiert werden. Die Erfinder haben das Differenzierungspotential in die adipogen, osteogenen und chondriogenen Linien evaluiert, wie zuvor beschrieben. In Kürze, für die adipogene und osteogene Differenzierung wurden MSCs in P1 mit einer Dichte von 1000 Zellen pro cm² ausplattiert und unter Standardkultivierungsbedingungen bis zum Erreichen der Subkonfluenz kultiviert. Anschließend wurde entweder die adipogene Differenzierung unter Verwendung des hMSC Adipogenetic BulletKit (PT-3004, Lonza) induziert, oder es wurde die osteogene Differenzierung unter Verwendung von "osteogenischem Medium" induziert, das zusammengesetzt war aus SCM mit 10⁻⁸ M Dexamethason, 0,2 mM Ascorbinsäure und 10 mM β-Glycerolphosphat (Sigma Aldrich). Nach drei Wochen unter Differenzierungsbedingungen wurden die Lipidvakuolen in den adipogenischen Kulturen mit Ölrot O und die Calciumablagerungen der osteogenischen Kulturen mit Alizarin Rot S gefärbt. Für die chondrogenische Differenzierung wurden 2,5x10⁵ hMSCs in P1 in Mikromassenpelletkulturen kultiviert. Die Differenzierung wurde unter Verwendung des hMSC chondrogenischen Differenzierungs-BulletKit (Lonza) induziert, versetzt mit TGF-Beta 3 (Lonza) als Wachstumsfaktor. Nach vierwöchiger Differenzierung wurden Gefrierschnitte der fixierten Pellets angefertigt und mit Safranin O oder Alcian blau gefärbt.

Um das charakteristische Oberflächenepitopmuster der hMSCs zu bestimmen, wurde eine durchflusszytometrische Analyse der Oberflächenmarkerexpression an hMSCs bei P1 mit einem FACScan (BD Biosciences) unter Verwendung der BD CellQuest Pro Software und (sekundären) PE-markierten Antikörpern (Anti-CD10, -CD14, -CD15, -CD19, -CD29, -CD31, -CD34, -CD 43, -CD44, -CD45, -CD56, -CD59, -CD71, - CD73, -CD90, -CD105, -CD106, -CD117, -CD130, -CD140a, -CD140b, -CD146, -CD166, -GD2, -HLA-Klasse I und -HLA-Klasse II (BD Biosciences); -CD93 (R&D Systems); - CD133 und -CD271 (Miltenyi Biotec); -CD243 (Millipore); -CD173 (AbD Serotec) und - W8B2 (BioLegend)) durchgeführt. PE-konjugierte oder nicht-markierte lgG1, -lgG2a und - lgM-Antikörper (BD Bioscience) wurden als Isotyp-abgestimmte Kontrollen verwendet. Der Sekundärantikörper war ein polyklonaler PE-konjugierter Anti-Maus lg der Ziege (BD Bioscience).

### 1.5 Behandlung von MSCs mit Polyethylenimin allein oder in Kombination mit Eisennanopartikeln

Die Behandlung der MSCs erfolgte in der Passage 2 bei einer Konfluenz von 90% für 4 Stunden in T-75-Flaschen. Am nächsten Tag wurden die MSCs für 4 Stunden mit dem kationischen linearen Polyethylenimin jetPEI™ (Polyplus-transfection, Illkirch, Frankreich) bei einer Endkonzentration von 10 µl/ml (PEI-MSC), oder mit einer Formulierung aus 10 µl/ml jetPEI™ plus 60 µg/ml Ferucarbotran (Resovist®, Bayer Schering AG, Berlin, Deutschland), bestehend aus superparamagnetischem EisenoxidNanopartikeln (mittlerer Partikeldurchmesser 4-6 nm), beschichtet mit Carboxydextran (mittlerer hydrodynamischer Durchmesser 60 nm) (PEI-NP-MSC). Während der Behandlung wurden die Zellen in dem Inkubator kultiviert (37°C, 5% feuchtes CO₂). Nach der Behandlung und vor der Ernte und Prozessierung für die Assays *in vitro* oder die Transplantation *in vivo* wurden die Zellen mit PBS gewaschen und für 24 Stunden in 15 ml Kultivierungsmedium kultiviert.

### 1.6 Durchflusszytometrische Analyse der Zielfindungsrezeptoren

Die durchflusszytometrische Analyse für die Evaluierung der Zielfindungsrezeptoren auf der Zelloberfläche der MSCs wurde mit einem FACSort Durchflusszytometer (BD Biosciences) unter Verwendung der BD CellQuest Pro Software durchgeführt. 24h nach der Behandlung wurden die Zellen mit Accutase™ (PAA Laboratories) abgelöst, gewaschen und in FACS-Puffer (PBS + 0,1% FBS) resuspendiert. Jede Probe enthielt eine Zellsuspension mit 2x10⁵ Zellen in 100 µl FACS-Puffer. 10 µl oder 20 µl (gemäß den Anweisungen des Herstellers) des FITC- oder PE-konjugierten Antikörpers wurden hinzugegeben. Nach einer Inkubationsdauer von 30 Minuten bei Raumtemperatur im Dunkeln und zwei Waschschritten konnte die Probe analysiert werden. Sämtliche Antikörper stammten von BD Biosciences oder R&D Systems. Die folgenden Antikörper wurden verwendet, um die Expression der Zelladhäsionsrezeptoren und ChemokinRezeptoren auf den unbehandelten MSCs zu untersuchen: Anti-human-α-1integrin (CD49a/VLA-1), -α-4integrin (CD49d/VLA-4), -β-1integrin (CD29), -H-CAM (CD44), -P-Selectin/LECAM-3 (CD62P), -endoglin (CD105), -VCAM (CD106), -CCR1 (CD191), - CCR4 (CD194), -CCR5 (CD195), -CCR6 (CD196), -CCR7 (CD197), -CXCR4 (CD184), -CXCR5 (CD185), -CXCR6 (CDw186/BONZO), und -CCR9 (CDw199). Die folgenden Antigene, die, wie sich herausgestellt hat, auf unbehandelten MSCs exprimiert werden, wurden verwendet, um die mit Polyethylenimin allein oder in Kombination mit Nanopartikeln behandelten MSCs weiter zu analysieren: Anti-human-α-1integrin (CD49a/VLA-1), -α-4integrin (CD49d/VLA-4), -β-1integrin (CD29/VLA-4), -H-CAM (CD44), -P-Selectin/LECAM-3 (CD62P), -endoglin (CD105), -VCAM (CD106), -CCR4 (CD194), - CXCR4 (CD184), und -CCR9 (CDw 199). Für Details der Fluoreszenzkonjugation und dem Subtyp/Isotyp der Immunglobuline, siehe nachstehende Tabelle 1.

**Tabelle 1: Antikörper für die durchflusszytometrische Analyse der Zielfindungsrezeptoren**

| **Spezifität** | **Fluoreszenz** | **Subtyp/Isotyp Immunglobuline** | **Bezugsquelle** |
|---|---|---|---|
| CD29 | FITC | IgG₁ | Serotec |
| CD44 | FITC | IgG₁ | R&D |
| CD49a | PE | IgG₁ | BD |
| CD49d | PE | IgG₁ | R&D |
| CD49e | PE | IgG₁ | R&D |
| CD62P | FITC | IgG₁ | R&D |
| CD105 | FITC | IgG₁ | R&D |
| CD106 | FITC | IgG₂ₐ | R&D |
| CCR1 | FITC | IgG_{2b} | R&D |
| CCR4 | PE | IgG_{2b} | R&D |
| CCR5 | PE | IgG_{2b} | R&D |
| CCR6 | PE | IgG_{2b} | R&D |
| CCR7 | FITC | IgG₂ₐ | R&D |
| CCR9 | PE | IgG₂ₐ | R&D |
| CXCR4 | FITC | IgG₂ₐ | R&D |
| CXCR5 | PE | IgG_{2b} | R&D |
| CXDR6 | PE | IgG_{2b} | R&D |

Für die Analyse wurde das geometrische Mittel der Fluoreszenzintensität verwendet. Um die Antigenexpression auf behandelten und unbehandelten MSCs zu vergleichen, wurde die Antigenexpression auf unbehandelten MSC auf 100% gesetzt. Sämtliche Daten wurden als relative Werte gemäß den jeweils unbehandelten MSCs der gleichen MSC-Population analysiert.

### 1.7 Bestimmung der Lebensfähigkeit und Durchmesser der MSCs

Die Lebensfähigkeit und der Durchmesser der abgelösten Zellen wurde mit einem CASY®2-Zellzählgerät und -analysesystem, Model TT (Roche Diagnostics, Mannheim, Deutschland) gemäß der ECE-Methode untersucht.

### 1.8 T-Zell-Proliferationsassays

Die immunsuppresiven Eigenschaften von PEI-MSCs, PEI-NP-MSCs und unbehandelten MSCs wurden in Co-Kultivierungsassays mit aktivierten allogenen T-Zellen analysiert. MSCs wurden durch 30-minütige Behandlung mit 40 µg/ml Mitomycin bei 37°C in serumfreiem Medium mitotisch inaktiviert. Anschließend wurden die MSCs dreimal in Kultivierungsmedium gewaschen, gezählt und in Platten mit 96 Vertiefungen im Dreifachansatz bei einer Dichte von 2x10⁴ Zellen pro Vertiefung ausplattiert. Nach 24 Stunden wurden 1x10⁵ mononukleare Zellen aus dem peripheren Blut (PBMNC), frisch isoliert mittels Dichtegradientenzentrifugation aus heparinisiertem Blut von gesunden Spendern, zu den jeweiligen Vertiefungen der BM-MSC-Kulturen hinzugegeben. Die T-Zellen wurden mit 10 µg/ml) Phytohaemagglutinin-L (Sigma-Aldrich) stimuliert und für 72 Stunden unter Standardkultivierungsbedingungen kultiviert. Während der letzten sechs Stunden wurden 100 µM Bromodeoxyuridin (BrdU) Markierungslösung aus dem kolorimetrischen Kit Cell Proliferation ELISA, BrdU (Roche) zu den (Co-)Kulturen hinzugegeben und die Zellproliferation wurde mittels ELISA unter Verwendung des Anti-BrdU-Antikörpers aus dem Kit gemäß den Anweisungen des Herstellers bestimmt. Die photometrische Lichtabsorption wurde in einem Plattenlesegerät gemessen und die Absorptionswerte wurden gemittelt und gegenüber der Kontrolle mit nur PBMNC des jeweiligen Blutspenders normalisiert.

### 1.9 Immunzytochemie in vitro

Für die Immunfluoreszenzfärbung von CXCR4 *in vitro* wurden die MSCs kultiviert und mit PEI oder PEI-NP in Kammerobjektträgern (Becton Dickinson) behandelt. Die Zellproben wurden jeweils für 30 Minuten bei 37°C mit dem Primärantikörper gegen CXCR4 (R&D Systems) inkubiert, gefolgt von einer Inkubation mit dem sekundären fluorochrom-markierten Antikörper (Alex Fluor 488, Invitrogen). Die Proben wurden in Eindeckmedium enthaltend 4',6-diamidino-2-phenylindol (DAPI) zur Kernfärbung eingebettet und mit einem Fluoreszenzmikroskop (Zeiss, Oberkochen, Deutschland) und der AxioVision Rel. 4.8 Software (Zeiss) untersucht.

### 1.10 RNA-Isolierung und qRT-PCR-Analyse für die CXCR4- und CD44-mRNA-Expression

RNA aus PEI-MSCs, PEI-NP-MSCs und aus unbehandelten MSCs wurde gemäß den Anweisungen des Herstellers unter Verwendung des RNeasy Mini Kit (Qiagen, Hilden, Deutschland) isoliert. Die verbleibende genomische DNA wurde unter Verwendung des RNase-Free DNase Set (Qiagen) verdaut. Die RNA-Konzentration wurde unter Verwendung eines NanoDrop Photometers (Thermo Scientific) bestimmt. Die RNA wurde bei -80°C für bis zu drei Monaten gelagert. Zur Analyse durch quantitative PCR wurden von jeder Probe 500 µg RNA unter Verwendung des Transcriptor First Strand cDNA Synthese Kits (Roche) gemäß den Anweisungen des Hersteller reverstranskribiert. Die resultierende cDNA wurde bei -20°C für bis zu 6 Monate gelagert. Für die Analyse der Expression der CXCR4 mRNA, CD44 mRNA und GAPDH mRNA als Haushaltsgen wurde eine PCR-Analyse der cDNA unter Verwendung von "ready-to-use"-Amplifizierungsprimergemische fürdie RT-PCR (Search-LC, Heidelberg, Deutschland) in Kombination mit LightCycler FastStart DNA Master SYBR Green I (Roche) und dem LightCycler Instrument (Roche) gemäß den Anweisungen des Herstellers durchgeführt. Die Ergebnisse der PCR wurden durch Normalisierung der Expression von jedem Zielgen in Bezug auf die Expression des Haushaltsgens GAPDH in jeder Probe analysiert.

### 1.11 Western Blot-Analysen

Das Western Blotting erfolgte unter Verwendung von Proteinlysaten aus behandelten und unbehandelten MSCs. Die Proteinkonzentrationen wurden nach dem Verfahren von Lowry bestimmt. Für jede Spur wurden 100 µg Protein einer Natriumdodecylsulfat-Polyacrylamid-Gelelektrophorese in einem 12,5%-igen Gel durchgeführt und durch Tankblotting auf PVDF-Membranen transferiert. Die Membranen wurden in 0,66% (v/w) I-Block oder 5% w/v nicht fettiger Trockenmilch (Tropix, Applied Biosystems, Weiterstadt, Deutschland) in PBST oder TBST (0,05% Tween 20 in PBS oder TBS, pH 7,4) für 1,5 Stunden blockiert und anschließend bei 4°C über Nacht mit Antikörpern gegen CXCR4 (1:500, AbD Serotec, Oxford, Vereinigtes Königreich), und CD44-Antikörpern, verdünnt (1:1000, Cell Signaling Technology, Danvers MA, USA) und GAPDH (1:1000, Markierungskontrolle, Chemicon International; Nürnberg, Deutschland) inkubiert. Für die Visualisierung der Antikörperbindung wurden die Membranen für 2 Stunden bei Raumtemperatur mit einem alkalische-Phosphatase-konjugiertem Anti-Kaninchen Antikörper aus der Ziege oder Maus als sekundärem Antikörper (Santa Cruz, Biotechnology, Kalifornien, USA), verdünnt 1:10.000 in I-Block, inkubiert und anschließend an CDP-Star (Tropix) als Chemolumineszenzsubstrat für eine Stunde im Dunkelraum exponiert. Die Signalintensitäten wurden unter Verwendung eines CCD-Kamerasystems aufgezeichnet.

### 1.12 Durchflusskammerassays

In allen Experimenten wurden als adhärente Schicht humane aortale endotheliale Zellen (hAoECs) (Promocell, Heidelberg, Deutschland, Katalog-Nr. C-12272) verwendet. Die HAoECs wurden bei 37°C in einer 5%-igen CO₂-Atmosphäre in Endothelzellmedium enthaltend Nutrientmischung F-12 (HAM) (Gibco Invitrogen) und Dulbecco's Mod Eagle Medium (DMEM) (Gibco Invitrogen) in einem Verhältnis von 4:1 kultiviert. Das Medium wurde mit 30 µg/ml Endothelzellwachstumszusatz (BD, Heidelberg), 50 µg/ml Heparin (Sigma), 100 µg/ml Streptomycin und 100 IE/ml Penicillin (Biochrom, Berlin, Deutschland) und 20% FBS (PAA) versetzt. Das Medium wurde alle drei bis vier Tage gewechselt.

HAoECs wurden auf Ibidi-Mikroträger (µ-Slide I (0,4) Luer, Collagen IV, IBIDI, München) mit einer Dichte von 7,2x10⁵ Zellen/cm² ausplattiert und über Nacht inkubiert, was zu einer konfluenten, adhärenten Schicht führte. Die HAoECs wurden mit 2,5 ng/ml TNFa (R&C, Wiesbaden, Deutschland) für 16 Stunden bei 37°C aktiviert.

Behandelte oder unbehandelte MSCs (P4 oder P5) wurden gewaschen, mit Trypsin abgelöst, gezählt und auf eine Konzentration von 3x10⁵ Zellen/ml eingestellt. Die Mikroträger wurden in einen Durchfluss-basierten Adhäsionsassay inkorporiert. Zusammengefasst, eine Spritzenpumpe (PHD 2000, Harvard Apparatus, Natick, MA) bewegte die Zellsuspension in Medium durch die Mikroträger mit einer kontrollierten Rate von 0,1 ml/min, wobei die Visualisierung der Zellen in dem Träger durch Phasenkontrastmikroskopie (Axiovert, Zeiss, Oberkochen, Deutschland) möglich war. Die MSCs wurden für 10 Minuten durch die Mikroträger perfundiert. Für die Analyse der MSC-Adhäsion unter Scherstress-Bedingungen wurden Videosequenzen (10 Sekunden) mit einer Digitalkamera (CF15 MC, Kappa, Gleichen, Deutschland) aufgezeichnet. Eine Zellbewegung von weniger einem Zelldurchmesser innerhalb von 10 Sekunden wurde als adhärent definiert. Um Geschwindigkeiten zu messen, wurden Aufnahmen dieser Videosequenzen bei 25 Frames/Sekunden digitalisiert, und die Entfernung der Bewegung der MSCs während des 10-Sekunden-lntervalls wurde in Pixel umgerechnet. Sämtliche Messungen erfolgten unter Verwendung einer selbsterstellten Software zur Bilderkennung (CellTracker, C. Zanke, Universitätsklinikum, Tübingen).

Um den Einfluss der Oberflächenantigene auf die Adhäsion zu evaluieren, führten die Erfinder Blockierungsexperimente mit geeigneten Antikörpern durch. Die MSCs wurden mit 10 µg/ml Anti-CD44-Antikörpern (BD, Heidelberg, Deutschland) oder Anti-CXCR4-Antikörpern (R&D Systems, Wiesbaden, Deutschland) für 30 Minuten behandelt. Ovarzellen aus dem chinesischen Hamster (CHO) wurden als Negativkontrollen verwendet.

### 1.13 Tiermodell zur Hirnschädigung

Sprague-Dawley-Ratten (Charles River, Wilmington, MA) (Gewicht zu Beginn der Experimente: 220 - 300 g) wurden unter konstanten klimatischen Bedingungen in einem Tierhalteraum mit 12:12 Stunden Hell-Dunkel-Zyklus gehalten. Sämtliche Experimente wurden in der Lichtphase durchgeführt. Die Ratten hatten Zugang zu 15 g Standardrattenfutter pro Tag und Wasser nach Belieben. Nach der Ankunft vom Anbieter hatten sämtliche Ratten zumindest 10 Tage Zeit, sich vom Transportstress zu akklimatisieren und zu erholen.

Für die unilaterale Läsion des Hippocampus wurden die Tiere entweder mit Pentobarbital (64 mg/kg) oder Isofluran (1-2%) in Luft mit perioperativer Fentanylanästhesie (3-5 µg/kg s.c.) anästhetisiert. Die post-chirurgische Analgesiebehandlung erfolgte durch Injektionen von Carprofen (täglich 50 mg/kg s.c.). Der Kopf eines jeden Tieres wurde in einem stereotaktischen Rahmen (Stoelting, Dublin, Irland) fixiert, die Haut auf dem Schädel wurde für die Desinfektion mit lodinlösung behandelt und es erfolgte ein kurzer Einschnitt durch die Haut. Es wurden kleine Löcher in die distinkte stereotaktische Lokalisierung für den Hippocampus gebohrt, ohne die Dura Mater zu beschädigen. Die Mikroinjektion (60 mmol/µl) von Quinolinat (Sigma-Aldrich) erfolgte mittels einer Mikrospritze von Hamilton (Kanülendurchmesser 0,45 mm) über eine Minute in den linken Hippocampus (Koordinaten relativ zu dem Bregma: AP -3,8 mm, L +2,4 mm, V -3,2 mm). Die Kanüle verblieb für weitere zwei Minuten in Position und wurde anschließend relativ langsam herausgezogen. Die Schädellöcher wurden vorsichtig mittels Knochenwachs verschlossen und die Haut wurde unter Verwendung von 2-4 Stichen verschlossen. Bei allen Ratten heilten die Wunden ohne Komplikationen innerhalb von 2-3 Tagen nach dem Eingriff.

5x10⁶ behandelte (PEI oder PEI-NP) oder unbehandelte GFP⁺MSCs wurden in 800 µl steriler phosphatgepufferter Saline resuspendiert und 7 Tage nach dem chirurgischen Eingriff durch intravenöse Injektion in die laterale Schwanzvene unter kurzer Ketamin-Anästhesie verabreicht (100-125 mg/kg i.p.). Die Anzahl der Tiere pro Gruppe betrug: 5 (unbehandelte MSCs; Immunhistochemie), 5 (PEI-MSCs; Immunhistochemie); 6 (PEI-NP-MSCs; Immunhistochemie); 12 (keine Zellen; 6 mit Läsion, 6 ohne Läsion; Multiplex).

Die Präparation des Gewebes erfolgte 24 Stunden nach der Zellverabreichung nach Enthauptung des jeweiligen Tiers unter Verwendung einer Guillotine nach Ketamin-Anästhesie. Die linke und rechte Lunge und beide Gehirnhälften wurden entnommen und für die immunhistochemischen und Multiplexanalysen, wie unten beschrieben, eingefroren.

Von den linken und rechten Gehirnhälften der Ratten wurden sagittale Gefrierschnitte (20 µm Dicke) hergestellt. Zur Detektion von GFP⁺MSCs wurden die Schnitte mit 4',6-Diamidino-2-phenylindol (DAPI) (Vector Laboratories Burlingame, CA) gefärbt. Für die Quantifizierung der GFP⁺MSCs wurden in regelmäßigen Intervallen Sagittalschnitte angefertigt, beginnend mit einer interhemisphärischen Fissur, und durch Fluoreszenzmikroskopie unter Verwendung eines Olympus BX51 Mikroskops (Olympus, Hamburg, Deutschland) und der "AnalySIS"-Software (Soft Imaging System GmbH, Deutschland) analysiert. Die Zahlen der GFP⁺MSCs wurden vom gesamten Bereich des Hippocampus erhalten.

Längliche Cryoschnitte (25 µm Dicke) der gesamten linken Lunge wurden in regelmäßigen Intervallen angefertigt und entweder mit DAPI für die GFP⁺MSC Quantifizierung eingebettet oder für die Immunzytochemie verarbeitet, wie unten beschrieben. Von 5 Feldern pro Lungenschnitt erfolgte die Quantifizierung von GFP⁺MSCs (30 Schnitte pro Organ).

Gehirne von den Kontrollratten (keine Zelltransplantation, die mit Quinolinsäure lädiert wurden, wurden entfernt, unmittelbar eingefroren und bei -80°C bis zur Analyse gelagert. Die Gehirne wurden entlang der interhemisphärischen Fissur geteilt; die linke und rechte Hemisphäre wurden gewogen und separat analysiert. Eiskalter Lysepuffer (300 mM NaCl, 50 mM Tris, 2 mM MgCl₂, 0,05% Nonidet P-40), enthaltend eine Tablette "Complete Protease Inhibitor" (Roche Diagnostics) wurde in einem Verhältnis von 1/3 (Gewicht/Volumen) hinzugegeben. Das Gewebe wurde auf Eis durch 15 Stöße in einem Glas-Glas-Homogenisator getrennt, welcher mit 1200 rpm rotierte. Anschließend wurden die Homogenate durch Zentrifugation für 10 Minuten bei 1500 x g geklärt, und der Überstand wurde in Aliquote geteilt und bei -80°C eingefroren. Die Quantifizierung des Proteingehalts erfolgte unter Verwendung des BIO-Rad DC Protein Assays (München, Deutschland). Die Bestimmung der Rattenzytokine (Monozyten-chemotaktisches Protein [MCP]-1, Interferon [IFN]-γ, Tumornekrosefaktor [TNF]-α, RANTES (reguliert und normal T-Zell-exprimiert und sekretiert), Interleukin [IL]-1α, IL-1β, IL-6, IFN-γ-induziertes Protein [IP]-10 [CXCL 10], MCP-3 und makrophageninflammatorisches Protein [MIP]-1a), erfolgte unter Verwendung eines Multiplex-Bead-Immunoassay-Systems (Procarta Zytokine Assay Kit; Affymetrix, Inc., Santa Clara, CA) gemäß den Anweisungen des Herstellers. Die Ergebnisse werden als pg/ml (oder pg/mg Protein) dargestellt.

### 1.14 Statistische Analyse

Die statistische Analyse erfolgte unter Verwendung der ANOVA-Analyse der Varianz oder des zweiseitigen t-Tests. Unterschiede wurden als signifikant gewertet bei p<0,05.

### 2. Ergebnisse

### 2.1 Prinzip der Zielfindung von MSC

In der Fig. 1 ist das Prinzip der Zielfindung von MSCs dargestellt. Die Funktion der Rezeptoren, welche die Adhäsion an Endothel in der Lunge (A) bzw. in nichtentzündlich verändertem Gewebe/Blutgefäßen (B) vermittelt, wird durch PEI blockiert. Hierdurch können die MSCs diese Bereiche ungehindert über den Blutstrom passieren. Im Zielgebiet (C) sind die Chemokine verstärkt exprimiert. Da PEI die Funktion der Chemokinrezeptoren nicht beeinträchtigt, können die MSCs dort ungehindert einwandern. In der Summe können mehr PEI-behandelte MSCs als unbehandelte MSCs in das Zielgebiet einwandern, da diese in geringerem Ausmaß in der Lunge bzw. in nicht entzündlich verändertem Gewebe/Blutgefäßen zurückgehalten werden.

### 2.2 Basischarakterisierung der humanen BM-MSCs

Analysiert durch Durchflusszytometrie, zeigten die MSCs eine positive Expression von CD10, CD29, CD44, CD59, CD73, CD90, CD105, CD140b, CD146, CD166 und GD2, zeigten jedoch keine Expression von CD14, CD34, CD45 und CD133 (Daten nicht gezeigt). Nach der Behandlung mit den jeweiligen Differenzierungsmedien, differenzierten die MSCs in Zellen der adipogenen, osteogenen und chondrogenen Linie *in vitro.* Ferner beeinflusste die Behandlung mit Polyethylenimin allein oder in Kombination mit Nanopartikeln nicht das Differenzierungspotential der MSCs *in vitro* (Fig. 2A, C, E).

### 2.3 Lebensfähigkeit und Durchmesser der MSCs

Die Lebensfähigkeit der MSCs wurde durch die Behandlung mit Polyethylenimin allein oder in Kombination mit Nanopartikeln nicht beeinflusst. Der prozentuale Anteil der lebensfähigen Zellen betrug 90,5% (±0,9% SA) für unbehandelte MSCs, 88,2% (±1% SA) für PEI-MSCs und 88,0% (±1,9% SA) für PEI-NP-MSCs (Fig. 3A).

Der mittlere Durchmesser der PEI-MSCs war geringfügig, jedoch signifikant geringer (20,2 µm [±0,6 µm SA]) als der Durchmesser der unbehandelten MSCs (21,4 µm [±0,7 µm SA]) oder PEI-NP-MSCs (22,0 µm [±0,5 µm SA]) (Fig. 3B).

### 2.4 Immunmodulatorische Kapazität der MSCs

Die immunmodulatorische Kapazität der MSCs wurde über die Fähigkeit der MSCs, die Proliferation von aktivierten T-Zellen *in vitro* zu unterdrücken, bestimmt. Die Proliferation von 1x10⁵ T-Zellen, aktiviert durch PHA, konnte signifikant durch die Zugabe von 0,1x10⁵ MSCs oder 0,2x10⁵ MSCs unterdrückt werden, wohingegen die Zugabe von 0,05x10⁵ MSCs oder 0,025x10⁵ MSCs keine Auswirkung auf die Proliferation von T-Zellen zeigte. Es konnten keine Unterschiede zwischen der immunmodulatorischen Kapazität von unbehandelten MSCs, PEI-MSCs oder PEI-NP-MSCs beobachtet werden (Fig. 4).

### 2.5 Muster der Zielfindungsrezeptoren und Chemokinrezeptoren auf den MSCs

Unbehandelte MSCs zeigten keine Expression von CCR1 (CD191), CCR5 (CD195), CCR6 (CD196), CCR7 (CD197), CXCR5 (CD185) und CXCR6 (CDw186/BONZO) *in vitro* (Daten nicht gezeigt). Die Behandlung der MSCs mit PEI allein resultierte in einem verminderten Nachweis von H-CAM, Endoglin, VCAM-1 und VLA-1 über die Durchflusszytometrie. Im Vergleich mit unbehandelten MSCs erhöhte sich der Nachweis von CCR4 nach einer Behandlung mit PEI allein. Im Gegensatz hierzu, resultierte die Behandlung von MSCs mit PEI in Kombination mit Nanopartikeln in einer erhöhten Detektion von VCAM-1 und CXCR4 im Vergleich zu unbehandelten MSCs und MSCs, die mit PEI allein behandelt wurden. Im Vergleich zu unbehandelten MSCs nahm der Nachweis von VLA-1 nach Behandlung mit PEI in Kombination mit Nanopartikeln ab. Die Behandlung der MSCs mit PEI allein oder in Kombination mit Nanopartikeln führte zu keiner Veränderung des Nachweises von P-Selectin, VLA-4 (CD49d, β-1-Integrin) und CCR9 im Vergleich zu unbehandelten MSCs (Fig. 5).

### 2.6 Fluoreszenzmikroskopie in vitro

Die Gesamtexpression von CXCR4 wurde durch die Behandlung der MSCs mit Polyethylenimin allein nicht beeinflusst und es konnte eine gleichmäßige Verteilung der CXCR4-Moleküle auf der Oberfläche der unbehandelten MSCs und der MSCs, die mit PEI allein behandelt wurden, beobachtet werden (Fig. 6A, B). Hierzu im Gegensatz, war das CXCR4-Signal stark erhöht auf MSCs, die mit PEI in Kombination mit Nanopartikeln behandelt wurden. Insbesondere zeigten die CXCR4-Moleküle ein aggregiertes Verteilungsmuster auf der Zelloberfläche von PEI-NP-MSCs (Fig. 6C). PEI bilden Komplexe mit Nanopartikeln auf der Oberfläche von MSCs, bevor eine Inkorporation in die Zellen durch Endozytose erfolgt. Die Erfinder konnten zeigen, dass die erhöhte CXCR4-Expression in engem Zusammenhang mit der Lokalisierung der PEI-NP-Komplexen auf der Oberfläche der MSCs steht (Fig. 7A-C). Kontrollexperimente zum Isotyp konnten eine unspezifische Autofluoreszenz der Nanopartikel ausschließen (Fig. 7D).

### 2.7 Expression der CD44 mRNA und CXCR4-mRNA in vitro

Die Behandlung von MSCs mit PEI allein resultierte in einer erhöhten Expression der CD44 mRNA im Vergleich zu unbehandelten MSCs oder MSCs, die mit PEI in Kombination mit Nanopartikeln behandelt wurden (Fig. 8A). Eine Behandlung der MSCs mit PEI allein oder in Kombination mit Nanopartikeln hatte keine signifikante Wirkung auf die Expression der mRNA des CXCR4-Transkriptes, Variante 1 (Fig. 8B). Bemerkenswerterweise exprimierten humane MSCs keine mRNA des CXCR4-Transkriptes, Variante 2 (Daten nicht gezeigt).

### 2.8 Expression des CD44-Proteins und CXCR4-Proteins in vitro

Die Behandlung der MSCs mit PEI allein oder in Kombination mit Nanopartikeln resultierte in einer erhöhten Expression des Gesamtproteins von CD44 im Vergleich zu unbehandelten MSCs. Die Behandlung der MSCs mit PEI allein resultierte in einer verminderten Expression des gesamten Proteins von CXCR4 im Vergleich zu unbehandelten MSCs oder MSCs, die mit PEI in Kombination mit Nanopartikeln behandelt wurden (Fig. 8C).

### 2.9 Adhäsionskapazität der MSCs an Endothelzellen unter Scherstress-Bedingungen in vitro

Unter dynamischen Scherstress -Bedingungen verminderte die Behandlung der MSCs mit PEI allein die Adhäsionskapazität der MSCs an Endothelzellen, die mit TNFa aktiviert waren, während die Behandlung der MSCs mit Nanopartikeln plus PEI die Adhäsionskapazität der MSCs erhöhte (Fig. 9A). Bei nicht-aktivierten Endothelzellen, war die Adhäsionskapazität von unbehandelten MSCs und PEI-NP-MSCs im Vergleich zur Adhäsionskapazität an aktivierten Endothelzellen signifikant reduziert und kaum irgendeine MSC konnte an CHO-Zellen detektiert werden, die an TNFa exponiert waren (Fig. 9B, C), was darauf hinweist, dass die Adhäsion von unbehandelten MSCs und PEI-NP-MSCs an Endothelzellen von der Interaktion von intakten Adhäsionsmolekülen abhängt. Interessanterweise war die Adhäsionskapazität von MSCs, die mit PEI allein behandelt wurden, an aktivierte Endothelzellen nicht unterschiedlich im Vergleich zu nicht-aktivierten Endothelzellen (Fig. 9D). Die Adhäsionskapazität von unbehandelten MSCs und PEI-NP-MSCs konnte signifikant durch die Zugabe von blockierenden Antikörpern gegen CD44 bzw. CXCR4 (Fig. 9E, F, H, I) reduziert werden, wohingegen die Adhäsionskapazität von MSCs, die mit PEI allein behandelt wurden, durch die Zugabe von blockierenden Antikörpern gegen CD44 oder CXCR4 nicht reduziert werden (Fig. 9G, J).

### 2.10 Zielfindung der MSCs in vivo

Das Zielfindungsmuster von unbehandelten MSCs, PEI-NP-MSCs und PEI-MSCs wurde nach intravenöser Verabreichung vom MSCs in einem Rattenmodell zur unilateralen Hippocampus-Gehirnschädigung evaluiert. Um die periphere Zielfindungsrate in einem nicht-geschädigten parenchymalen Organ zu analysieren, wurden GFP-positive MSCs in den Lungen der Tiere identifiziert und quantifiziert. Korrelierend zu der reduzierten Adhäsionsrate *in vitro* konnten signifikant weniger MSCs, die mit PEI allein behandelt wurden, in den Lungen im Vergleich zu unbehandelten MSCs und PEI-NP-MSCs detektiert werden. Die Zielfindungsrate der PEI-NP-MSCs in den Lungen zeigte einen zunehmenden Trend, wobei jedoch kein statistisch signifikanter Unterschied im Vergleich zu unbehandelten MSCs beobachtet werden konnte (Fig. 10).

Nach der unilateralen Verabreichung von Quinolinsäure in den Hippocampus-Bereich konnten signifikant mehr MCP-1a, MIP-1a und IP-10 in der lädierten Hemisphäre im Vergleich zur nicht-lädierten Hemisphäre detektiert werden (Daten nicht gezeigt). Es wurden keine Unterschiede in den Konzentrationen von MCP-3, IL-1a, RANTES und TNF-α beobachtet (Daten nicht gezeigt), und es waren keine relevanten Mengen von IL-1b, IL-6 und IFN-γ nachweisbar (Daten nicht gezeigt).

Im Gehirn konnten signifikant mehr MSCs und PEI-MSCs im lädierten Hippocampus im Vergleich zum nicht-lädierten (kontralateralen) Hippocampus detektiert werden. Die Anzahl der PEI-MSCs, die als Ziel den lädierten Hippocampus finden konnten, war hochsignifikant erhöht im Vergleich zur Anzahl der unbehandelten MSCs oder PEI-NP-MSCs in dem lädierten Hippocampus, was auf eine zielgerichtete Migration der PEI-MSCs an die Stelle der Schädigung hindeutet (Fig. 11A). Interessanterweise konnte kein Unterschied in der Anzahl der PEI-NP-MSCs im lädierten Hippocampus im Vergleich zum nicht-lädierten Hippocampus beobachtet werden. Es konnten keine Unterschiede im Zielfindungsmuster der unbehandelten MSCs, PEI-MSCs und PEI-NP-MSCs in den nicht-lädierten Bereichen des Gehirns (Lateralventrikel, Kortex, Striatum) sowie dem Gehirn insgesamt (Fig. 11B-E) detektiert werden.

### 3. Fazit

Die Erfinder stellen ein einfach durchzuführendes Verfahren zur Verbesserung der Zielfindungskapazität von Stammzellen bereit.

## Patentansprüche

1. In vitro-Verfahren zur Verbesserung der Zielfindungskapazität von mesenchymalen Stammzellen (MSCs), das folgende Schritte aufweist:
1. Bereitstellung von MSCs, und
2. Inkubation der MSCs mit einem linearen kationischen Polymer.

2. Verfahren nach Anspruch 1, **dadurch gekennzeichnet, dass** das lineare kationische Polymer Polyethylenimin (PEI) ist.

3. Verfahren nach einem der vorherigen Ansprüche, **dadurch gekennzeichnet, dass** die MSCs humane MSCs sind.

4. Verfahren nach einem der vorherigen Ansprüche, **gekennzeichnet, durch** folgenden weiteren Schritt:
3. Inkubation der MSCs mit Eisenoxidnanopartikeln.

5. Verfahren nach Anspruch 4, **dadurch gekennzeichnet, dass** die Eisenoxidnanopartikel Ferucarbotran sind.

6. Verfahren nach einem der vorherigen Ansprüche, **dadurch gekennzeichnet, dass** die Verbesserung der Zielfindungskapazität durch Inhibition der Zelladhäsionsrezeptoren der MSCs erfolgt.

7. Verfahren nach einem der vorherigen Ansprüche, **dadurch gekennzeichnet, dass** die Verbesserung der Zielfindungskapazität durch Stimulation der Chemokinrezeptoren der MSCs erfolgt.

8. In vitro-Verwendung eines linearen kationischen Polymers zur Verbesserung der Zielfindungskapazität von mesenchymalen Stammzellen (MSCs).

9. Verwendung nach Anspruch 8, **dadurch gekennzeichnet, dass** das lineare kationische Polymer Polyethylenimin (PEI) ist.

## Claims

1. *In vitro* method to improve the homing capacity of mesenchymal stem cells (MSCs) comprising the following steps:
1. providing MSCs, and
2. incubating the MSCs with a linear cationic polymer.

2. Method of claim 1, **characterized in that** the linear cationic polymer is polyethylenimine (PEI).

3. Method of any of the previous claims, **characterized in that** the MSCs are human MSCs.

4. Method of any of the previous claims, **characterized by** the following further step:
3. incubating the MSCs with ferric oxide nanoparticles.

5. Method of claim 4, **characterized in that** the ferric oxide nanoparticles are ferucarbotran.

6. Method of any of the previous claims, **characterized in that** improving the homing capacity is realized by an inhibition of the cell adhesion receptors of the MSCs.

7. Method of any of the previous claims, **characterized in that** improving the homing capacity is realized by a stimulation of the chemokine receptors of the MSCs.

8. *In vitro* use of a linear cationic polymer to improve the homing capacity of mesenchymal stem cells (MSCs).

9. Use of claim 8, **characterized in that** the linear cationic polymer is polyethylenimine (PEI).

## Revendications

1. Procédé *in vitro* servant à améliorer la capacité de ciblage de cellules souches mésenchymateuses (CSM), qui présente des étapes suivantes :
1) fourniture de CSM, et
2) incubation des CSM avec un polymère cationique linéaire.

2. Procédé selon la revendication 1, **caractérisé en ce que** le polymère cationique linéaire est une polyéthylèneimine (PEI).

3. Procédé selon l'une quelconque des revendications précédentes, **caractérisé en ce que** les CSM sont des CSM humaines.

4. Procédé selon l'une quelconque des revendications précédentes, **caractérisé par** l'étape supplémentaire suivante :
3) incubation des CSM avec des nanoparticules d'oxyde de fer.

5. Procédé selon la revendication 4, **caractérisé en ce que** les nanoparticules d'oxyde de fer sont du ferucarbotran.

6. Procédé selon l'une quelconque des revendications précédentes, **caractérisé en ce que** l'amélioration de la capacité de ciblage est effectuée par inhibition des récepteurs d'adhésion de cellules des CSM.

7. Procédé selon l'une quelconque des revendications précédentes, **caractérisé en ce que** l'amélioration de la capacité de ciblage est effectuée par stimulation des récepteurs de chimiokine des CSM.

8. Utilisation *in vitro* d'un polymère cationique linéaire pour améliorer la capacité de ciblage de cellules souches mésenchymateuses (CSM).

9. Utilisation selon la revendication 8, **caractérisée en ce que** le polymère cationique linéaire est une polyéthylèneimine (PEI).
